Europäisches Patentamt

European Patent Office

Office européen des brevets

(11) Publication number: **0 138 974**

**B1**

(12) **EUROPEAN PATENT SPECIFICATION**

(45) Date of publication of patent specification: **16.08.89**

(51) Int. Cl.⁴: **A 61 M 29/02**

(21) Application number: **84901547.4**

(22) Date of filing: **23.03.84**

(86) International application number:
**PCT/US84/00455**

(87) International publication number:
**WO 84/03633 27.09.84 Gazette 84/23**

(54) **INVERTED BALLOON CATHETER HAVING SEALED THROUGH LUMEN.**

<table>
<tr><td>

(30) Priority: **25.03.83 US 478708**

(43) Date of publication of application:
**02.05.85 Bulletin 85/18**

(45) Publication of the grant of the patent:
**16.08.89 Bulletin 89/33**

(84) Designated Contracting States:
**AT BE CH DE FR GB LI LU NL SE**

(56) References cited:
**US-A-3 253 594**
**US-A-3 409 016**
**US-A-4 271 839**
**US-A-4 324 262**
**US-A-4 403 612**

</td><td>

(73) Proprietor: **FOGARTY, Thomas J.**
**770 Welch Road Suite 201**
**Palo Alto, CA 94304 (US)**

(72) Inventor: **FINN, James, C., III**
**Hulme 8H Escondido Village**
**Stanford, CA 94305 (US)**
Inventor: **KINNEY, Thomas, B.**
**541 El Medio Avenue**
**Mountain View, CA 94040 (US)**
Inventor: **FOGARTY, Thomas J.**
**770 Welch Road Suite 201**
**Palo Alto, CA 94304 (US)**

(74) Representative: **Schmitz, Jean-Marie et al**
**OFFICE DENNEMEYER S.à.r.l. P.O. Box 1502**
**L-1015 Luxembourg (LU)**

</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention concerns a dilatation catheter comprising a catheter body and a balloon attached to the distal end of said body and inverted within said body, means including a tubular extension being attached to said balloon to define a through lumen. The invention also comprises a method of providing a dilatation catheter of the evertible-invertable balloon type with a sealed through lumen to enable the body of said catheter to be moved along a guide wire while maintaining a sealed balloon system.

In the dilatation catheter art, it is old to provide such catheters with evertable-invertable balloon elements. Attention is directed to US—A—4 254 774 in this regard. Such balloon catheters, however, have not, insofar as we are aware, been provided with sealed through lumens enabling the passage through the balloons of guide wire and other instruments.

US—A—4271839 describes a balloon catheter wherein the balloon is inverted within the distal end of the catheter. In one embodiment of said balloon catheter a tubular extension is attached to the balloon defining a through lumen.

The dilation catheter of the present invention is characterized in that closure means in the form of an elastomeric plug is provided within said lumen to maintain said balloon in sealed condition for eversion and subsequent radial expansion, said closure means being penetrable by a guide wire, thin cannular, or other thin element without loss of the sealed condition of said balloon.

The method for providing the dilation catheter of the present invention comprises the steps of attaching a balloon to the distal end of the catheter body, initially forming said balloon with an axial opening at its distal end, sealing said opening with an elastomeric plug which has been formed with a normally closed axial passageway through said plug to accommodate a guide wire while said balloon system remains sealed.

The essential object of the invention is to provide a balloon catheter of the evertable-invertible type with a means for passing an object, such as a guide wire or a cannular, through the balloon while maintaining a sealed balloon system.

When the balloon is subjected to inflation pressure the plug acts as an imperforate part of the balloon whether or not a guide wire or the like is extending through the plug.

In order that the invention may be fully understood reference is made on the accompanying drawings wherein:

Fig. 1 is a view in perspective of a preferred embodiment of the catheter of the invention.

Fig. 2 is an enlarged view in diametral cross-section of said catheter.

Fig. 3 is a similar view showing the catheter strung on a guide wire and positioned adjacent a stenosis to be treated.

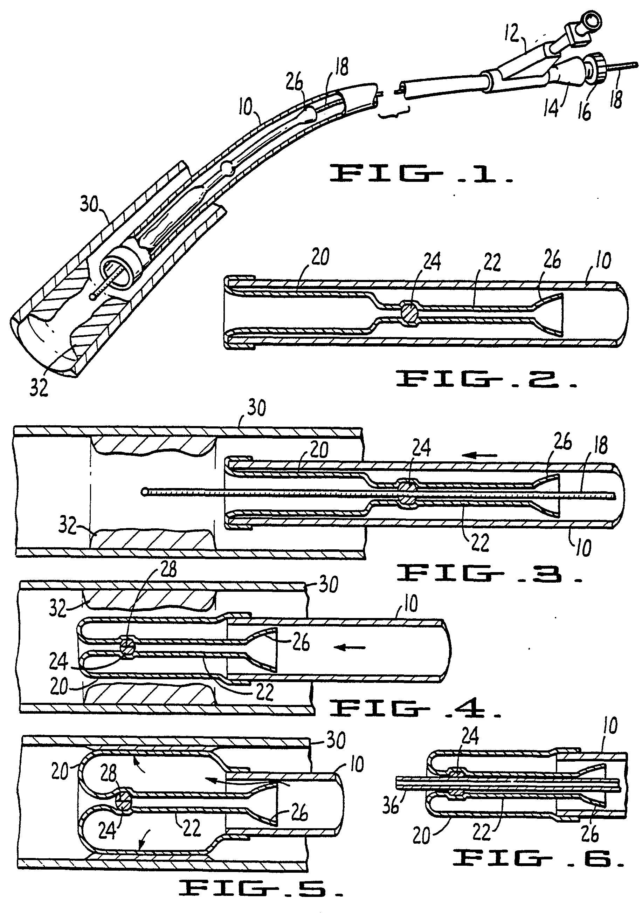

Fig. 2 is a similar view showing the balloon everted and within the stenosis.

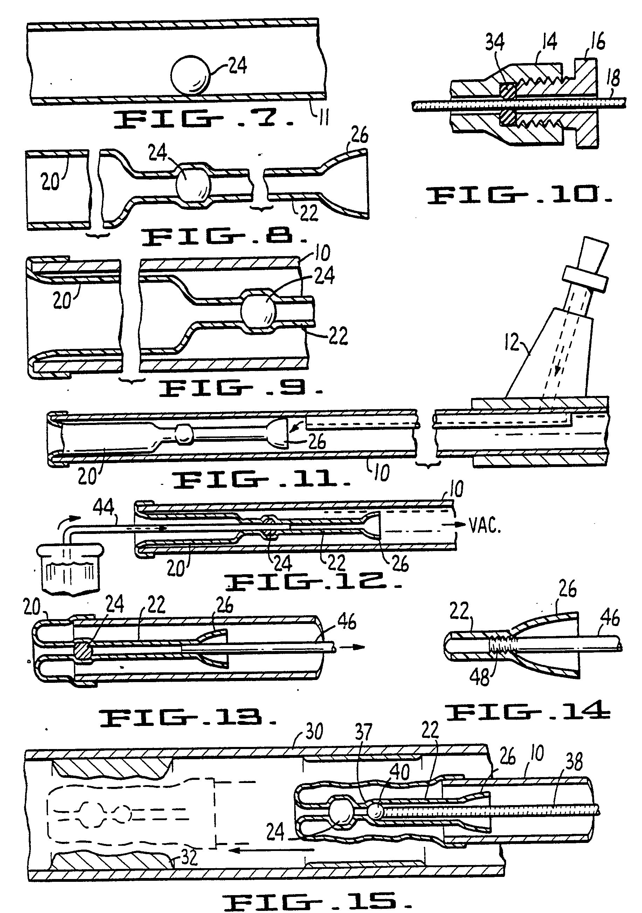

Fig. 5 is a similar view showing the balloon inflated to dilate the stenosis.

Fig. 6 is a similar view showing an open ended tube extending along the lumen of the catheter.

Fig. 7 is a view of tube and plug elements prior to their securement together, the tube being shown in diametral section.

Fig. 8 is a view of the balloon and plug assemblage prior to the attachment of the assemblage to the catheter.

Fig. 9 shows the assemblage of Fig. 8 in attached relation to the catheter and in inverted position therein.

Fig. 10 is a detailed view in diametral cross-section of a sealing means which may be employed at the proximal end of the catheter to maintain the balloon pressurized while a guide wire extends through the catheter.

Fig. 11 is a view showing a form of purging system which may be employed with the catheter.

Fig. 12 shows another form of purging system which may be employed with the catheter.

Fig. 13 illustrates a means for inverting the balloon.

Fig. 14 shows a modified form of balloon inversion means.

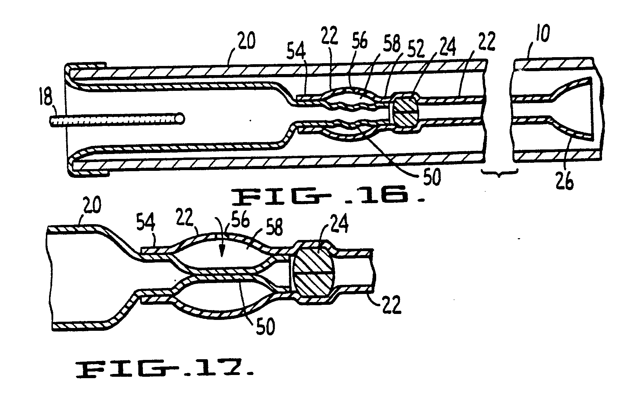

Fig. 15 shows how the catheter may be moved from one stenosis to another without inverting the balloon.

Fig. 16 is a view in diametral section of another embodiment of the catheter.

Fig. 17 is an enlarged detail view illustrating how the lumen sealing means of this embodiment operates.

The catheter of Figs. 1—15 comprises a flexible tubular body 10, fluid feed fitting 12, hub 14, screw plug 16, guide wire 18, balloon 20, balloon tubular extension 22, plug 24 and funnel-like end 26 formed on extension 22. The end of the balloon is folded over the distal end of tube 10 and sealingly secured thereto.

Figs. 7—9 illustrate the preferred way of making the balloon and balloon extension assemblage. The plug 24 (Fig. 7) of elastomeric material, such as silicone adhesive/sealer, is pierced axially with a needle which is then withdrawn, thereby forming a normally closed or sealed passageway 28 through the seal. The plug is then placed within a tube 11 of polyethylene or other suitable material for an expansible non-elastic balloon. By heat treatment the tube is caused to shrink in the region surrounding and adjacent to the plug so as to form the shape of the assemblage shown in Fig. 8. The plug is gripped and embraced by the containing tube, or extension 22. The assemblage is then secured to the tube 10, as shown in Fig. 9.

30 is an artery or other body tube having a stenosis or stricture 32. The sequence of usage of the catheter to accomplish dilatation of the stenosis 32 is shown in Figs. 3—5. The catheter is threaded along guide wire 18 which traverses the plug passageway 28 and the entire catheter. When the distal end of the catheter is positioned adjacent stenosis 32, the guide wire is preferably withdrawn from the balloon and balloon extension assemblage 20, 22. The wire may remain in the proximal end of the catheter to serve as part of a sealing means (Fig. 10) comprising wire 18, compressible O-ring 34 and screw plug 16 to compress ring 34 into sealing and gripping relation with wire 18.

Inflation fluid is then injected through fitting 12 to cause eversion of the balloon into stenosis 32, as shown in Fig. 4. After balloon eversion, the balloon is further inflated to expand radially and dilate the stenosis, as shown in Fig. 5. During all this inflation activity, the plug passageway 28 remains in its normally closed position. The balloon and balloon extension are thereby provided with a sealed through lumen.

Regardless of the condition of the balloon, whether it is inverted, everted or radially inflated, thin, tubular elements, such as 36 in Fig. 6, may be passed through the plug. The tube 36 may be then used to monitor pressure conditions at the distal tip of the catheter; it may be used for the delivery of diagnostic or therapeutic substances to the distal end of the catheter; it may be used for the sampling of body fluids, such as blood; and it may be used for the passage of instruments or tools through the catheter, such as temperature monitors or fiber optic cords.

Fig. 15 illustrates how the catheter may be moved from one stenosis to another without bothering to first invert the balloon. For this purpose the extension 22 is preferably provided with a step 36. A stylet 38 having a round or flat tip 40 is inserted into the extension to abut the step. The catheter tube or body 10 and stylet 38 are then jointly moved to position the everted balloon within the next adjacent stenosis 32.

Figs. 11 and 12 illustrate two purging systems for the catheter. In Fig. 11 the catheter is provided with a purge lumen through which purge liquid is passed to the interior of the catheter through fitting 12. When the catheter has been filled with the purge liquid, issuance of liquid through a bleeder valve, not shown, indicates that all of the air has been purged from the catheter. In Fig. 11 the purge liquid is introduced into the catheter through a needle-like fitting 44 which extends through plug passageway 28. Coupling of the proximal end of the catheter to a vacuum source causes the catheter to be filled with purge fluid.

Figs. 13 and 14 illustrate a retraction means to re-invert the balloon. Extension 22 may have tube 46 frictionally fitted within the end thereof. When the tube 46 is pulled to the right, the balloon becomes re-inverted. As shown in Fig. 14, the tube or rod 46 may have a threaded end 48. The corresponding threads in extension 22 may be pre-formed or formed by self-threading.

A further embodiment of the catheter is shown in Figs. 16—17. Between the plug 24 and balloon 20, extension 22 encloses a length 24 of elastomeric tubing 50. One end of this tubing is bonded to the extension 22 in the region 52. The other end of tubing 50 may be integral with the end of the balloon 20. Extension 22 is bonded to the balloon in the region 54. Extension 22 is provided with aperture 56 in overlying relation to tube 50. When inflation liquid is introduced into the catheter, the liquid enters chamber 58 between extension 22 and the tube 50 to compress the latter and close the passage along tube 50, as illustrated in Fig. 17. Further application of inflation liquid causes eversion of the balloon, and still further application of inflation liquid produces radial expansion of the balloon. The longitudinal passage through tube 50 remains closed under the effect of inflation liquid pressure during all of these operations.

In the embodiment of Figs. 16 and 17, the guide wire 18 may be passed through the sealed through lumen in either direction, while in the embodiment of Figs. 18—20 such passage must be made from the distal end of the catheter in order to avoid collapsing of the balloon 23.

It is to be pointed out that the elastomeric plug 24 of the embodiments of Figs. 1—17 can be fixed in place in various ways. The preferred way, as disclosed, is to shrink the containing tube partially around the ends of the plug, thereby mechanically bracing the plug in position. The plug can be adhesively secured to the containing tube, either with or without the mechanical bracing relationship between tube and plug.

All of the above-described embodiments allow placement of the catheter body over a guide wire which has previously been manipulated into the desired lumen or space. This provides important advantages over previous linear extrusion catheters which are incapable of being used directly over a guide wire and must be positioned with a guiding catheter, if that is possible, or without any guide or aid at all. The expertise which physicians have in placing guide wires, particularly in the cardiovascular and urological systems, makes this wire-compatibility feature of particular importance.

The catheter embodiments of Figs. 1—17 allow replacement of the guide wire after balloon extrusion, or eversion, if desired, simply by pushing the guide wire back through the seal and past the limit of balloon eversion and then by withdrawing the catheter over the guide wire (i.e., guide wires or other thin objects may be passed in either direction through the seal). The physician can thereby easily maintain guide wire access to a difficultly reached lumen or space if further procedures or measurements are required in that space or another space which is reached through it. The present catheter provides these advantages while maintaining the advantages of the linear extrusion catheter.

## Claims

1. A dilatation catheter comprising a catheter body (10) and a balloon (20) attached to the distal end of said body and inverted within said body, means including a tubular extension (22) being attached to said balloon (20) to define a through lumen, characterized in that closure means in the form of an elastomeric plug (24) is provided within said lumen to maintain said balloon (20) in sealed condition for eversion and subsequent radial expansion, said closure means being penetrable by a guide wire (18), thin cannular, or other thin element without loss of the sealed condition of said balloon.

2. The catheter of claim 1, further characterized in that said extension has a funnel-like element (26) at its distal end to guide said wire, thin cannular, or other thin element into said lumen.

3. The catheter of claim 1 or 2 further characterized in that said plug (24) has a normally closed, self-sealing axial passageway (28) therein adapted to yieldingly accommodate said wire (18), thin cannular, or other thin element without loss of the sealed condition of said balloon.

4. The catheter of claim 3, further characterized in that said plug (24) fits within a radially enlarged portion of said extension (22) and is secured in place between radially diminished portions of said extension.

5. The catheter of claim 3, further characterized in that said closure means comprises an elastomeric sleeve (50) within said extension (22), said sleeve (50) having one end fixedly attached to said balloon (20) and the other end fixedly attached to said plug (24), said extension (22) having an aperture (56) which provides communication for the application of balloon-eversion fluid pressure to an annular chamber (58) between said extension (22) and said sleeve (50) to thereby collapse said sleeve (50) upon itself and seal said lumen against fluid flow.

6. The catheter of claim 1, further characterized in that said tubular extension (22) has its proximal end secured to said balloon (20) and has its distal end closure by said plug (24) except for a self-sealing normally closed passageway (28) extending through the plug (24), said tubular extension being collapsible upon itself upon the application thereto of balloon-eversion fluid pressure to seal said lumen against fluid flow.

7. A method of providing a dilatation catheter of the evertable-invertable balloon type with a sealed through lumen to enable the body of said catheter to be moved along a guide wire while maintaining a sealed balloon system, comprising the steps of attaching a balloon (20) to the distal end of the catheter body (10), initially forming said balloon (20) with an axial opening at its distal end, sealing said opening with an elastomeric plug (24) which has been formed with a normally closed axial passageway (28) through said plug (24) to accommodate a guide wire (18) while said balloon system remains sealed.

**Patentansprüche**

1. Dilatationskatheter mit einem Katheterkörper (10) und einem Ballon (20), der an dem distalen Ende des Körpers befestigt und in den Körper eingestülpt ist, wobei eine Einrichtung mit einem rohrförmigen Fortsatz (22) an dem Ballon (20) befestigt ist, um einen durchgehenden Hohlraum zu bilden, dadurch gekennzeichnet, daß eine Verschlußeinrichtung in Form eines elastomeren Pfropfens (24) in dem Hohlraum vorgesehen ist, um den Ballon (20) in abgedichtetem Zustand zum Ausstülpen und anschließenden radialen Aufweiten zu halten, wobei die Verschlußeinrichtung von einem Führungsdraht (18), einer dünnen Kanüle oder einem anderen dünnen Element durchdringbar ist, ohne daß der abgedichtete Zustand des Ballons verlorengeht.

2. Katheter nach Anspruch 1, weiter dadurch gekennzeichnet, daß der Fortsatz ein trichterartiges Element (26) an seinem distalen Ende zum Führen des Drahtes, der dünnen Kanüle oder des anderen dünnen Elements in dem Hohlraum hat.

3. Katheter nach Anspruch 1 oder 2, weiter dadurch gekennzeichnet, daß der Pfropfen (24) einen normalerweise geschlossenen, sich selbst abdichtenden axialen Durchlaß (58) aufweist, der den Draht (18), die dünne Kanüle oder das andere dünne Element nachgiebig aufnehmen kann, ohne daß der abgedichtete Zustand des Ballons vorlorengeht.

4. Katheter nach Anspruch 3, weiter dadurch gekennzeichnet, daß der Pfropfen (24) in einen radial erweiterten Teil des Fortsatzes (22) paßt und zwischen radial verkleinerten Teilen des Fortsatzes festgehalten wird.

5. Katheter nach Anspruch 3, weiter dadurch gekennzeichnet, daß die Verschlußeinrichtung eine elastomere Hülse (50) in dem Fortsatz (22) aufweist, wobei ein Ende der Hülse (50) an dem Ballon (20) und das andere Ende an dem Pfropfen (24) fest angebracht ist, wobei der Fortsatz (22) eine Öffnung (56) hat, die eine Verbindung herstellt für die Beaufschlagung einer ringförmigen Kammer (58) zwischen dem Fortsatz (22) und der Hülse (50) mit Ballonausstülpfluiddruck, um dadurch die Hülse (50) in sich zusammenzudrükken und den Hohlraum gegen eine Fluidströmung abzudichten.

6. Katheter nach Anspruch 1, weiter dadurch gekennzeichnet, daß der rohrförmige Fortsatz (22) mit seinem proximalen Ende an dem Ballon (20) befestigt ist und sein distales Ende durch den Pfropfen (24) verschlossen hat, mit Ausnahme eines sich selbst abdichtenden, normalerweise geschlossenen Durchlasses (28), welcher sich durch den Pfropfen (24) erstreckt, wobei der rohrförmige Fortsatz, wenn er mit Ballonausstülpfluiddruck beaufschlagt wird, in sich zusammendrückbar ist, um den Hohlraum gegen eine Fluidströmung abzudichten.

7. Verfahren zum Herstellen eines Dilatationskatheters vom Typ mit ausstülpbarem/einstülpbarem Ballon, mit einem abgedichteten durchgehenden Hohlraum, damit der Körper des Katheters längs eines Führungsdrahtes bewegt werden kann, während ein abgedichtetes Ballonsystem erhalten bleibt, beinhaltend die Schritte Befestigen eines Ballons (20) an dem distalen Ende des Katheterkörpers (10), anfängliches Versehen des Ballons (20) mit einer axialen Öffnung an seinem distalen Ende, Verschließen der Öffnung mit einem elastomeren Pfropfen (24), der mit einem normalerweise verschlossenen axialen Durchlaß (28) durch den Pfropfen (24) zum Aufnehmen eines Führungsdrahtes (18), während das Ballonsystem abgedichtet bleibt, versehen worden ist.

## Revendications

1. Cathéter à dilatation comprenant un corps (10) de cathéter, ainsi qu'un ballon (20) fixé à l'extrémité distale de ce corps, tout en étant retourné vers l'intérieur au sein de celui-ci, des moyens englobant un prolongement tubulaire (22) étant fixés à ce ballon (20) en vue de définir une lumiere de part en part, caractérisé en ce que l'on prévoit, au sein de cette lumière, un moyen de fermeture sous forme d'un bouchon (24) en matière élastomère, en vue de maintenir ce ballon (20) en état d'étanchement pour le retournement vers l'extérieur et la dilatation radiale ultérieure, ce moyen de fermeture pouvant être traversé par un fil métallique de guidage (18), une canule mince, ou tout autre élément mince, sans perte de l'état d'étanchement du ballon.

2. Cathéter selon la revendication 1, davantage caractérisé en ce que le prolongement comporte, à son extrémité distale, un élément (26) en forme d'entonnoir, en vue de guider le fil métallique, la canule mince, ou tout autre élément mince, dans la lumière.

3. Cathéter selon la revendication 1 ou 2, davantage caractérisé en ce que le bouchon (24) comporte en son sein un passage axial (28) autoétanche, normalement fermé, approprié à s'adapter au fil métallique (18), à la canule mince, ou à tout autre élément mince, en épousant leur forme, et sans perte de l'état d'étanchement du ballon.

4. Cathéter selon la revendication 3, davantage caractérisé en ce que le bouchon (24) s'ajuste au sein d'une partie radialement élargie du prolongement (22), tandis qu'il est fixé en place entre les parties radialement amincies du prolongement.

5. Cathéter selon la revendication 3, davantage caractérisé en ce que le moyen de fermeture comprend un manchon (50) en matière élastomére au sein du prolongement (22), ce manchon (50) comportant une extrémité fixée fermement au ballon (20), tandis que l'autre extrémité est fixée fermement au bouchon (24), le prolongement (22) comportant une ouverture (56), celle-ci permettant à l'application d'une pression de fluide, exercée, par le retournement du ballon vers l'extérieur, d'entrer en communication avec une chambre annulaire (58) ménagée entre le prolongement (22) et le manchon (50), permettant ainsi l'affaissement de celui-ci sur lui-même, tout en étanchant la lumière contre le courant de fluide.

6. Cathéter selon la revendication 1, davantage caractérisé en ce que l'extrémité proximale du prolongement tubulaire (22) est fixée au ballon (20), tandis que son extrémité distale est fermée par le bouchon (24), à l'exception d'un passage (28) auto-étanche et normalement fermé, s'étendant à travers le bouchon (24), le prolongement tubulaire pouvant s'affaisser sur lui-même, suite à l'application de la pression de fluide exercée par le retournement du ballon vers l'extérieur, afin d'étancher la lumière contre le courant de fluide.

7. Procédé en vue de munir un cathéter à dilatation du type à ballon pouvant se retourner vers l'intérier et vers l'extérieur, d'une lumière de part en part étanchée, en vue de permettre au corps de ce cathéter de se déplacer le long d'un fil métallique de guidage, tout en maintenant un système de ballon étanché, ce procédé comprenant les étapes consistant à fixer un ballon (20) à l'extrémité distale du corps (10) du cathéter, tout en ayant pratiqué, au préalable, une ouverture axiale à l'extrémité distale de ce ballon (20), étancher cette ouverture avec un bouchon (24) en matière élastomére, dans lequel on a pratiqué un passage axial (28) normalement ferme, en vue d'y adapter un fil métallique de guidage (18), tandis que le système à ballon demeure étanché.

EP 0 138 974 B1

FIG.1.

FIG.2.

FIG.3.

FIG.4.

FIG.5.

FIG.6.

FIG.7.

FIG.8.

FIG.9.

FIG.10.

FIG.11.

FIG.12.

FIG.13.

FIG.14

FIG.15.

FIG.16.

FIG.17.